# EUROPEAN PATENT SPECIFICATION

(11) **EP 2 654 853 B1**
(45) Date of publication and mention of the grant of the patent: **19.11.2014**
(21) Application number: 11813676.1
(22) Date of filing: 22.12.2011
(51) Int. Cl.: A61M 5/24, A61M 5/315, A61M 5/00, A61M 5/31, A61M 5/34

(54) **PISTON ROD DRIVE SYSTEM FOR A DRUG DELIVERY DEVICE AND DRUG DELIVERY DEVICE HAVING THE SAME**
KOLBENSTANGENANTRIEBSSYSTEM FÜR EINE WIRKSTOFFFREISETZUNGSVORRICHTUNG UND WIRKSTOFFFREISETZUNGSVORRICHTUNG MIT SOLCHEM KOLBENSTANGENANTRIEBSSYSTEM
SYSTÈME D'ENTRAÎNEMENT DE TIGE DE PISTON POUR UN DISPOSITIF D'ADMINISTRATION DE MÉDICAMENT ET DISPOSITIF D'ADMINISTRATION AVEC UN TEL SYSTÈME D'ENTRAÎNEMENT

(30) Priority: 23.12.2010 EP 10196790; 23.12.2010 US 201061426585 P
(43) Date of publication of application: 30.10.2013
(73) Proprietor: Novo Nordisk Health Care AG, 8050 Zürich (CH)
(72) Inventor: HANSEN, Søren, Kjellerup, DK-2880 Bagværd (DK)
(74) Representative: Vejgaard-Nielsen, Jeppe
(86) International application number: PCT/EP2011/073798
(87) International publication number: WO 2012/085208

(56) References cited:
- EP-A2- 0 937 471
- US-A- 6 004 298
- US-A1- 2008 071 226

## Description

### FIELD OF THE INVENTION

The present invention relates to a drive system for a piston rod. The drive system is adapted for use in a drug delivery device, particularly of the type which is capable of mixing substances to produce an administrable drug product.

### BACKGROUND OF THE INVENTION

Prefilled syringes containing a specific volume of medication when initially provided by the manufacturer are well known in the art of medical devices. Such syringes may e.g. comprise a reservoir barrel provided with a needle interface at one end and a piston at the other end. For transport and storage reasons the needle interface end is typically closed, e.g. by a tip cap or a penetrable rubber septum. In use, an operator mounts an injection needle on the needle interface end (whereby the rubber septum may be penetrated), inserts the needle at the desired injection site and empties, or partly empties, the syringe by slowly pressing the piston down through the barrel using an appertaining piston rod.

Some types of medication need to be on a specific form in order to secure an acceptable shelf life. For example, some products for treating bleeding disorders are lyophilised and stored as powders until just prior to administration. Dedicated mixing devices exist offering a fast and easy reconstitution of the lyophilised product using a suitable solvent, such as e.g. water. One such type of mixing device is the so-called dual chamber syringe. A dual chamber syringe conventionally comprises a reservoir barrel with an outlet end and two pistons, one of the pistons (the front piston) providing a separating wall effectively dividing the reservoir into two chambers, and the other piston (the rear piston) providing a seal. During storage the powder product is comprised in one of these chambers and the solvent is comprised in the other chamber. Typically, the solvent is comprised in the rear chamber between the two pistons, such that an operator during use of the device may transfer the solvent to the front chamber around the front piston, e.g. via a bypass channel, by pressing the rear piston towards the front piston. An example of such a dual chamber syringe is e.g. found in US 5,788,670 (Schott Glas). Another example of prior art devices is given in US 6 004 298. The preamble of claim 1 is based on the disclosure of said document.

A common drawback of both single chamber and dual chamber syringes is uneven, or jerky, piston motion due to stick-slip effects arising from the interaction between the piston material and the reservoir wall. This results in the user having less control of the speed with which the piston is advanced and may, in the case of a dual chamber syringe, cause a too fast transfer of solvent from one chamber to another, leading to an undesired foaming of the final mixed product.

The dual chamber syringe system Lyo-Ject^{®}, developed by Vetter, includes an elongated drug container, a connector piece and a piston rod with an outer screw thread along approximately the distal half of its length. The screw thread is adapted to mate with an inner thread in the connector piece to provide for a rotational advancement of the piston rod in the drug container during the reconstitution phase. Upon reconstitution the piston rod is advanced non-rotationally in the drug container.

The fact that the user is required to carry out a repetitive rotational motion rather than a linear depression in order to advance the piston rod during reconstitution eliminates the above described risk of too fast piston movement due to stick-slip effects. However, the helical advancement of the piston rod is transferred to a helical advancement of the rear piston, to which the piston rod is coupled, i.e. in addition to being slowly moved forward in the drug container, the piston rotates with respect the container wall. This entails an increased tendency of the liquid in the rear chamber to become dragged proximally along the peripheral surface of the rear piston as the pressure in the rear chamber rises, thereby causing leakage of the system.

Both in relation to storage and handling it is preferable that a dual chamber drug delivery device is relatively small, to save space and to ease the operation. In syringe systems like e.g. the aforementioned Lyo-Ject^{®} the piston rod may be adapted to be connected to the drug container right before use, thereby shortening the syringe during storage. However, the user must then himself assemble the two parts before taking the system into use, and in the pre-activation state, after assembly of the piston rod and the drug container, the system is at least about twice the length of the drug container because the piston rod must be able to advance the front piston all the way to the outlet to empty the container. Such a length may render the system difficult and/or slower to operate for some users.

### SUMMARY OF THE INVENTION

It is an object of the invention to provide a solution which eliminates, or at least reduces, drawbacks of the prior art.

In particular, it is an object of the invention to provide a drug delivery device with a piston rod drive system which ensures a both controllable and non-rotational advancement of a piston in the drug reservoir.

It is a further object of the invention to provide such a device which ensures a slow advancement of the piston during a first phase of use and enables a faster advancement of the piston during a second phase of use.

It is an even further object of the invention to provide a dual chamber type of drug delivery device which is relatively short and which does not require the user to couple the piston rod and the drug reservoir before use.

In the disclosure of the present invention, aspects and embodiments will be described which will address one or more of the above objects or which will address objects apparent from the below disclosure as well as from the description of exemplary embodiments.

In a first aspect of the invention a piston rod drive system is provided comprising a main body adapted for coupling with a reservoir, a user operable activation element operatively coupled with the main body and configured to move axially and rotationally relative to the main body between a first position and a second position, and a piston rod element operatively coupled with the activation element and the main body and configured to move axially and non-rotationally relative to the main body in response to the activation element being moved between the first position and the second position.

In the present context the term "user operable" is to be understood as directly user operable or user accessible, e.g. a user operable activation element is accessible to the user for manipulation thereof.

A rotational user operation of the activation element as required by the above piston rod drive system enables a slower and more controlled piston rod advancement compared to systems where a piston rod actuator is linearly depressed by e.g. the users thumb. The fact that the activation element is accessible to the user enables the provision of a smaller piston rod drive system than would be possible if additional mechanical and/or electronic components were to be incorporated for automatic actuation.

In particular embodiments, the activation element is further configured to move axially and non-rotationally relative to the main body between the second position and a third position. This enables, in a later phase, piston rod advancement by linear depression of the activation element instead of the slower rotational operation.

In one embodiment, the main body comprises a first inner engagement structure (e.g. a screw thread) and a second inner engagement structure (e.g. a key), and the user operable activation element comprises an outer engagement structure adapted to cooperate with the first inner engagement structure, and further an inner engagement structure. The piston rod element comprises a first outer engagement structure (e.g. a screw thread) adapted to cooperate with the inner engagement structure of the activation element, and a second outer engagement structure (e.g. a keyway) adapted to cooperate with the second inner engagement structure of the main body.

The piston rod element may further be configured to move axially and non-rotationally relative to the main body in response to the activation element being moved between the second position and the third position. In that case, and in relation to the above described embodiment, the second outer engagement structure may extend along the entire, or substantially entire, axial length of the piston rod element.

The main body may be provided with means for releasable coupling with a reservoir, or with a reservoir holder, or it may be adapted to provide a non-releasable engagement with a reservoir or with a reservoir holder. The piston rod element may be adapted to interact with, e.g. abut or engage, a piston in the reservoir when the reservoir or the reservoir holder is connected with the main body.

In another aspect of the invention a drug delivery device is provided comprising a drug reservoir and a piston rod drive system as described in the above operatively coupled with the drug reservoir. In such context, the above mentioned first position may e.g. correspond to a pre-activation state of the drug delivery device, the second position may e.g. correspond to a particular intermediate state of the drug delivery device, and the third position may e.g. correspond to an end-of-use state of the drug delivery device. The end-of-use state of the drug delivery device may particularly correspond to an end-of-content state of the reservoir, i.e. a state of the reservoir in which it has been emptied to the extent practically possible.

In particular embodiments, the piston rod drive system comprises a main body, a user operable activation element, and a piston rod, and the reservoir comprises an outlet, a proximal end portion, a front piston arranged between the outlet and the proximal end portion to provide a first chamber in the reservoir for a first substance, a rear piston arranged between the front piston and the proximal end portion to provide a second chamber in the reservoir for a second substance, and a transfer arrangement adapted to enable fluid transfer from one side of the front piston to the other, e.g. when the front piston is at a certain position in the reservoir. The main body is adapted to house at least a portion of the reservoir and it comprises a first inner thread structure and a key member. The user operable activation element comprises a sleeve portion which has a first outer thread structure, adapted to mate with the first inner thread structure, and a second inner thread structure. The piston rod, which is adapted to cause movement of at least the rear piston, comprises a second outer thread structure adapted to mate with the second inner thread structure (e.g. in a self-locking thread engagement) and an axially extending keyway adapted to cooperate with the key member.

In such a drug delivery device a rotational user manipulation of the activation element results in a non-rotational, axial advancement of the piston rod. Thereby, the user is prevented from performing an action that can lead to an overly fast piston displacement, while at the same time the rear piston is prevented from rotating relative to the reservoir wall.

In particular embodiments, the pitch of the second outer thread structure is greater than the pitch of the first inner thread structure. This has the effect that when the user turns the activation element the piston rod is displaced axially a greater distance relative to the main body than the activation element is. It thus requires relatively few revolutions of the activation element to move the rear piston towards the front piston and thereby execute a transfer of fluid from the second chamber to the first chamber.

The first inner thread structure may terminate in, or lead into, an axial groove such that when a leading face of the first outer thread structure travels to the end of the first inner thread structure it abuts a stop surface which prevents further rotation of the activation element. When this happens the activation element and the piston rod are in a specific relative axial position corresponding to the rear piston having been advanced into abutment, or non-releasable engagement, with the front piston. The rotational stop of the activation element provided by the stop surface marks the end of the fluid transfer phase, i.e. the drug delivery device provides a clear tactile feedback to the user of when the substances have been completely mixed.

The first outer thread structure may comprise a circumferentially limited thread segment which is capable of travelling the axial groove from the end of the first inner thread structure to an end-of-use position where the reservoir has been emptied. This will provide for a purely linear operation of the activation element during administration of the mixed product, and the user can therefore carry out this last part of the procedure using only one hand by simply pushing the activation element distally towards the main body in a manner well-known from conventional syringes.

The sleeve portion is structured to surround at least a portion of the piston rod and in one embodiment of the invention the sleeve portion comprises an opening in its axially extending wall. The piston rod is provided with a flexible arm, e.g. at its proximal end portion, which carries a latching protrusion having a configuration that allows it to be occupied in the wall opening of the sleeve. In the pre-activation state of the drug delivery device, and in the mixing phase, the flexible arm is biased radially inwards by the sleeve portion. The opening and the flexible arm are respectively positioned such that exactly when the leading face of the first outer thread structure abuts the stop surface the latching protrusion snaps into the opening, thereby providing an audible alert to the user that the liquid transfer phase is completed and further interlocking the activation element and the piston rod to ensure that all subsequent movements of the two are carried out in common.

By providing one or more automatic feedbacks to the user that the mixing phase is completed and the delivery phase can commence, the user is alerted to exactly when he has to change his mode of operation of the drug delivery device from rotation of the activation element to pure translation thereof.

The activation element may further comprise an enlarged knob member for interfacing with fingers or a hand of the user to enable easy manipulation of the piston rod drive system. The knob member may be provided with one or more arrows indicating the direction of operation to eliminate possible doubts as to which way to turn the activation element.

A drug delivery device as described in the present text is especially convenient for reconstitution of a powdered drug. However, the device is equally suitable for mixing of e.g. two liquids.

The present telescopic piston rod drive system enables the manufacturing of a considerably shorter device compared to devices which employ a conventional piston rod because a major portion of the piston rod, or if so desired the entire piston rod, can initially be housed in the activation element and can emerge axially therefrom during a first operational phase and co-translate with the activation element during a second operational phase. Thereby, the piston rod itself need not have a length comparable to the axial length of the reservoir to cause the front piston to move all the way through the reservoir to the outlet.

Particular examples of drugs which may be provided in powder form, e.g. lyophilised, include factor products, growth hormone, fertility drugs, and antibiotics.

The invention is further exemplified by the below clauses:
Clause 1. A piston rod drive system for a drug delivery device, the piston rod drive system comprising a) a main body comprising a1) a first engagement structure and a2) a second engagement structure, b) a user operable activation element comprising b1) a third engagement structure adapted to cooperate with the first engagement structure to enable a relative rotational motion between the main body and the activation element, the activation element further comprising b2) a fourth engagement structure, and c) a piston rod member defining a general axis and comprising c1) a fifth engagement structure adapted to cooperate with the fourth engagement structure to enable a relative rotational motion between the activation element and the piston rod member, and c2) a sixth engagement structure adapted to cooperate with the second engagement structure to enable a relative axial and non-rotational motion between the main body and the piston rod member.
   The piston rod drive system is configured such that when a user rotates the activation element relative to the main body the fifth engagement structure cooperates with the fourth engagement structure and the sixth engagement structure cooperates with the second engagement structure to cause an axial and non-rotational displacement of the piston rod member relative to the main body.
Clause 2. A system according to clause 1, wherein the first engagement structure comprises a first inner thread structure and the third engagement structure comprises a mating first outer thread structure.
   When the user rotates the activation element relative to the main body the activation element and the main body thus undergo a relative helical motion. The piston rod drive system may be configured such that the translational component of the displacement of the activation element relative to the main body has the same direction as the occasioned axial displacement of the piston rod member. Thereby, in case a portion of the activation element initially protrudes axially from the main body, the protrusion will be diminished during the first operational phase. This will provide a shortened system which is easier for the user to operate during the second operational phase.
Clause 3. A system according to clause 1, wherein the fourth engagement structure comprises a second inner thread structure and the fifth engagement structure comprises a mating second outer thread structure
Clause 4. A system according to clause 1, wherein the first engagement structure comprises a first inner thread structure and the third engagement structure comprises a mating first outer thread structure, and wherein the fourth engagement structure comprises a second inner thread structure and the fifth engagement structure comprises a mating second outer thread structure.
Clause 5. A system according to clause 4, wherein the pitch of the second outer thread structure is greater than the pitch of the first inner thread structure.
Clause 6. A system according to clause 4 or 5, wherein the second engagement structure comprises a key and the sixth engagement structure comprises a keyway.
Clause 7. A system according to any of clauses 4 - 6, wherein the first inner thread structure terminates in a stop surface structured to abut with a leading face of the first outer thread structure when the activation element and the piston rod member are brought to a specific relative axial position.
Clause 8. A system according to clause 7, wherein the stop surface constitutes a portion of an axial groove, and wherein the first outer thread structure comprises a circumferentially limited thread segment which is adapted to travel in the axial groove between the stop surface and an end-of-use position to displace the activation element non-rotationally relative to the main body.
Clause 9. A system according to clause 7 or 8, wherein the activation element comprises a sleeve member adapted to surround at least a portion of the piston rod member, and wherein the sleeve member comprises an opening and the piston rod member comprises a flexible arm with a latching protrusion structured to move into the opening when the activation element and the piston rod reach the specific relative axial position.
Clause 10. A system according to any of clauses 4 - 9, wherein the second inner thread structure and the second outer thread structure are in self-locking thread engagement (whereby relative axial motion between the activation element and the piston rod member is prevented during relative non-rotational axial motion between the activation element and the main body).
Clause 11. A drug delivery device comprising 1) a reservoir comprising 1a) an outlet, 1b) a proximal end portion, 1c) a first piston arranged between the outlet and the proximal end portion, 1d) a second piston arranged between the first piston and the proximal end portion, and 1e) a transfer arrangement adapted to enable fluid transfer from one side of the first piston to the other, and 2) a piston rod drive system according to any of clauses 1 - 10.
Clause 12. A device according to clause 11, wherein the first inner thread structure terminates in a stop surface, and wherein the pitch of the first inner thread structure and the pitch of the second outer thread structure are configured such that the piston rod member is displaced axially relative to the main body a distance corresponding to the second piston being brought to abutment or non-releasable engagement with the first piston when a leading face of the first outer thread structure is moved from a pre-activation position into abutment with the stop surface.

In the present specification, reference to a certain aspect or a certain embodiment (e.g. "an aspect", "a first aspect", "one embodiment", "an exemplary embodiment", or the like) signifies that a particular feature, structure, or characteristic described in connection with the respective aspect or embodiment is included in, or inherent of, at least that one aspect or embodiment of the invention, but not necessarily in/of all aspects or embodiments of the invention. It is emphasized, however, that any combination of features, structures and/or characteristics described in relation to the invention is encompassed by the invention unless expressly stated herein or clearly contradicted by context.

The use of any and all examples, or exemplary language (e.g., such as, etc.), in the text is intended merely to illuminate the invention and does not pose a limitation on the scope of the same, unless otherwise claimed. Further, no language or wording in the specification should be construed as indicating any non-claimed element as essential to the practice of the invention.

### BRIEF DESCRIPTION OF THE DRAWINGS

In the following the invention will be further described with references to the drawings, wherein
Figs. 1a-1c show cross-sectional views of a piston rod drive system according to an embodiment of the invention in different stages before and during use,
Fig. 2 shows an exploded perspective view of the piston rod drive system of Figs. 1a-1c,
Fig. 3 shows a perspective view of the piston rod drive system of Fig. 2, where half of the main body and the entire piston rod have been removed, and
Figs. 4a-4c show cross-sectional views of a drug delivery device according to an embodiment of the invention in different stages before and during use.

In the figures like structures are mainly identified by like reference numerals.

### DESCRIPTION OF EXEMPLARY EMBODIMENTS

When in the following relative expressions are used these refer to the appended figures and not necessarily to an actual situation of use. The shown figures are schematic representations for which reason the configuration of the different structures as well as their relative dimensions are intended to serve illustrative purposes only.

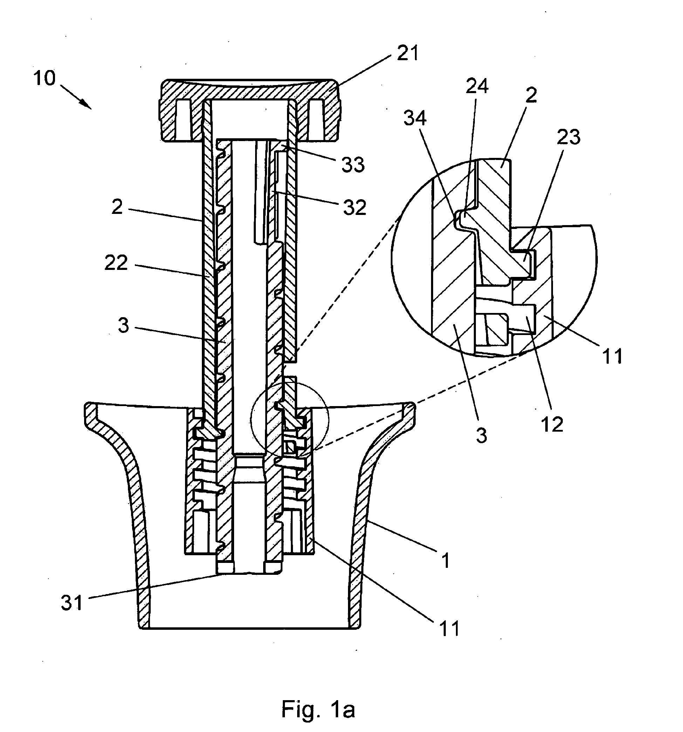

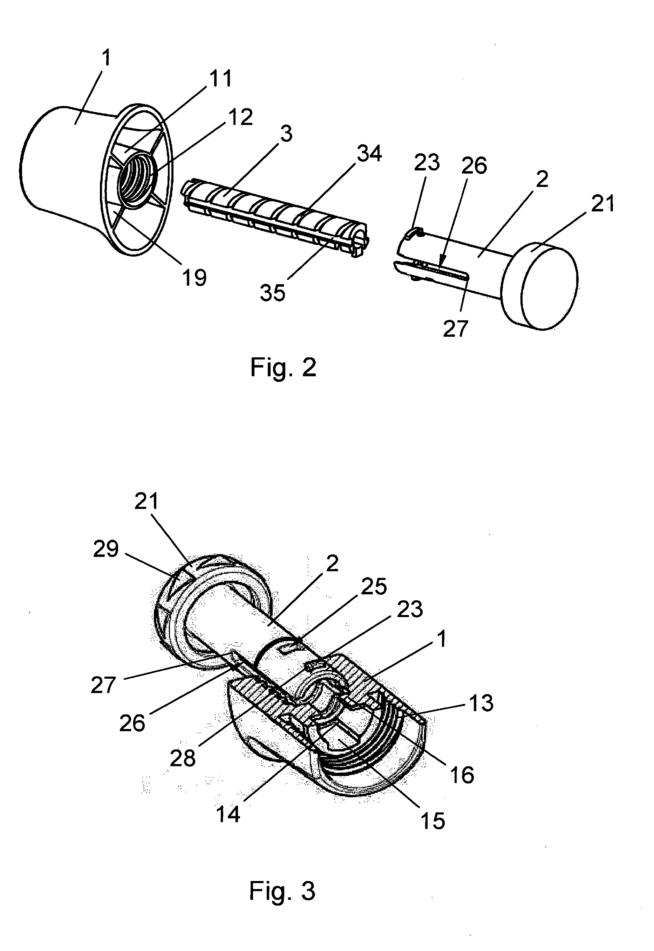

Figs. 1a-1c show cross-sectional views of a piston rod drive system 10 according to an embodiment of the invention in different states before and during use. The piston rod drive system 10 comprises three primary components, a main body 1, a driver 2, and a piston rod 3. The main body 1 has an outer hollow shell and an inner hub 11 provided with an interior female thread 12 along a proximal portion thereof. The driver 2 comprises a knob 21 for user manipulation and a sleeve 22 which at its distal portion is provided with a couple of exterior male thread segments 23 adapted to mate with the interior female thread 12. The sleeve 22 is further provided with interior male thread segments 24. The piston rod 3 is a hollow cylindrical structure which along its entire length is provided with an exterior female thread 34, adapted to mate with the interior male thread segments 24, as well as a couple of circumfer-entially opposite longitudinal keyways 35 (see Fig. 2) adapted for engagement with respective keys 16 (see Fig. 3) in the hub 11 to prevent rotation of the piston rod 3 relative to the main body 1. The piston rod 3 has a distal piston actuation end 31 which is adapted to interact with a piston (not shown) or an intermediate member like a piston rod foot (also not shown) coupled to the piston. At its proximal end portion the piston rod 3 is provided with a flexible finger 32 which terminates in a radial protrusion 33.

In Fig. 1a the piston rod drive system 10 is in a pre-use state, i.e. the driver 2 is at a proximal most position experiencing a maximum axial protrusion from the main body 1. Due to the configurations of the sleeve 22 and the protrusion 33 the flexible finger 32 is pre-tensioned in this state as it is deflected radially inwards by the inner sleeve wall. A rotational lock (not visible) prevents the driver 2 from becoming disengaged from the main body 1.

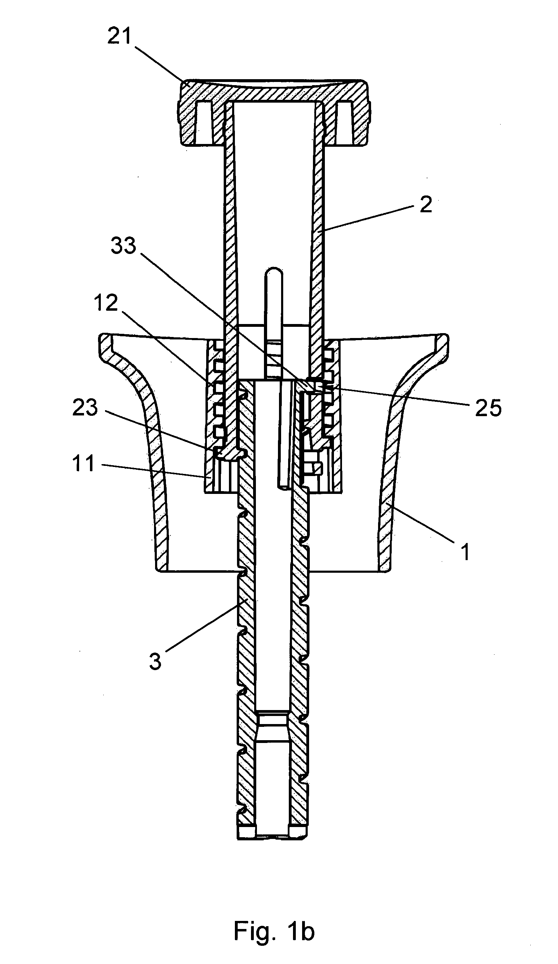

In Fig. 1b the exterior male thread segments 23 have travelled the interior female thread 12, in response to a user turning the knob 21 relative to the main body 1, and the leading segment is positioned at the end thereof. This has brought the knob 21 closer to the main body 1 and has further caused a helical advancement of the piston rod 3 inside the driver 2 to a point where an opening 25 in the sleeve 22 has allowed the flexible finger 32 to relax and thereby urge the protrusion 33 radially outwards into the opening 25. The piston rod drive system 10 is now in an operation transition state. The established engagement between the protrusion 33 and the opening 25 locks the piston rod 3 rotationally as well as translationally to the driver 2, thereby ensuring that all subsequent movements of the two are common. Because the piston rod 3 is rotationally locked with respect to the main body 1, the engagement between the protrusion 33 and the opening 25 further rotationally locks the driver 2 with respect to the main body 1. This ensures a purely translational subsequent distal motion of the driver 2 relative to the main body 1 as well as prevents proximal motion of the driver 2 relative to the main body 1 beyond this point.

It is noted that in this particular embodiment of the invention the pitch of the exterior female thread 34 is greater than the pitch of the interior female thread 12, which means that the axial travel of the piston rod 3 relative to the driver 2 from the pre-use state to the operation transition state is longer than the axial travel of the driver 2 relative to the main body 1. Thereby, a relatively long axial displacement of the piston actuation end 31 may be achieved in response to relatively few turns of the knob 21.

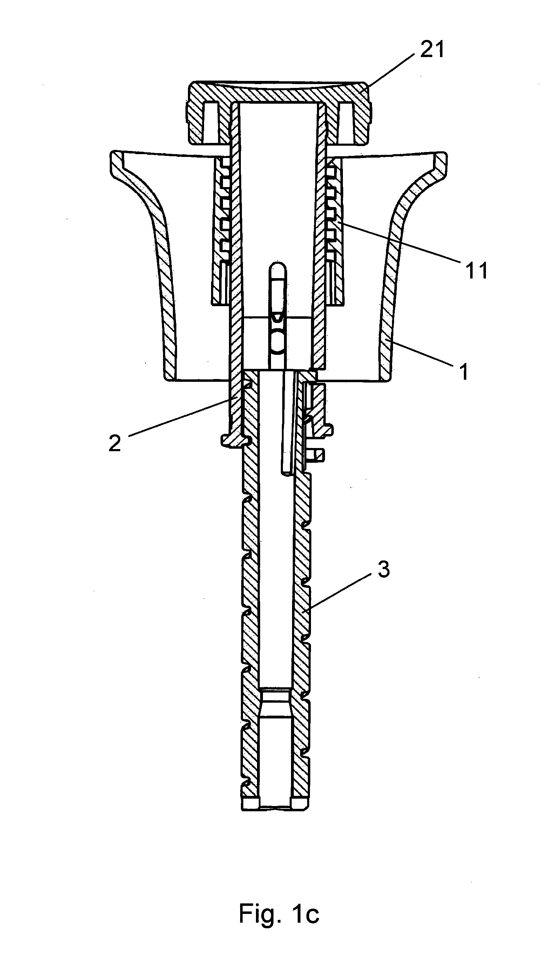

In Fig. 1c the piston rod 3 is fully advanced relative to the main body 1 and the piston rod drive system 10 is in an end-of-use state. The axial displacement of the driver 2 relative to the main body 1 from the operation transition state to the end-of-use state has been purely translational and has been caused by a user pushing the knob 21 distally towards the main body 1.

Fig. 2 is an exploded perspective view of the piston rod drive system 10 further detailing the three primary components. Especially, it is seen that the hub 11 is arranged concentrically in the main body 1, supported by a number of flanges 19, and that the longitudinal keyways 35 (only one is visible) extend along the entire length of the piston rod 3 and are superposed onto the exterior female thread 34. Furthermore, a couple of circumferentially opposite elongated slots 26 in the driver 2 (only one is visible) extend, respectively, from the proximal end of the sleeve 22 to a stop 27.

Fig. 3 is a perspective view of the main body 1 and the driver 2 showing an interaction between these two components in a situation of use. For the sake of clarity, in this figure only a portion of the main body 1 is shown and the piston rod 3 has been completely removed. The knob 21 is provided with circumferentially distributed arrows 29 indicating the direction of operation to the user. The relative position of the main body 1 and the driver 2 corresponds to a state of the piston rod drive system 10 just before the operation transition state. Another half turn (approximately) of the knob 21 in the direction of the arrows 29 will make the exterior male thread segments 23 travel the last portion of the interior female thread 12 and bring a leading face 28 into abutment with a rotational stop surface 14. Thereby, the operation transition state is reached and the user must shift his mode of operation from a rotational manipulation of the knob 21 to an axial depression thereof.

At the point where the leading face 28 is brought into abutment with the rotational stop surface 14 the elongated slots 26 align with the respective keys 16 in the hub 11 and all further movements of the driver 2 relative to the main body 1 are guided by the keys 16 and a pair of axial grooves 15 (only one is visible) for the respective exterior male thread segments 23. The maximum distal displacement of the driver 2 relative to the main body 1 from the operation transition state to the end-of-use state is defined by the position of the stops 27 on the sleeve 22. When the keys 16 reach the stops 27 the piston rod 3 has been fully extended in the piston rod drive system 10.

The interior wall of the main body 1 is in this embodiment provided with a screw thread 13 which is adapted to mate with a corresponding thread on a cartridge, or on a holder for a cartridge. In other embodiments, the main body may be adapted to be press-fitted onto a cartridge, or a cartridge holder, glued or otherwise attached thereto. In particular embodiments, the cartridge is pre-mounted in the main body when the system is offered to the user.

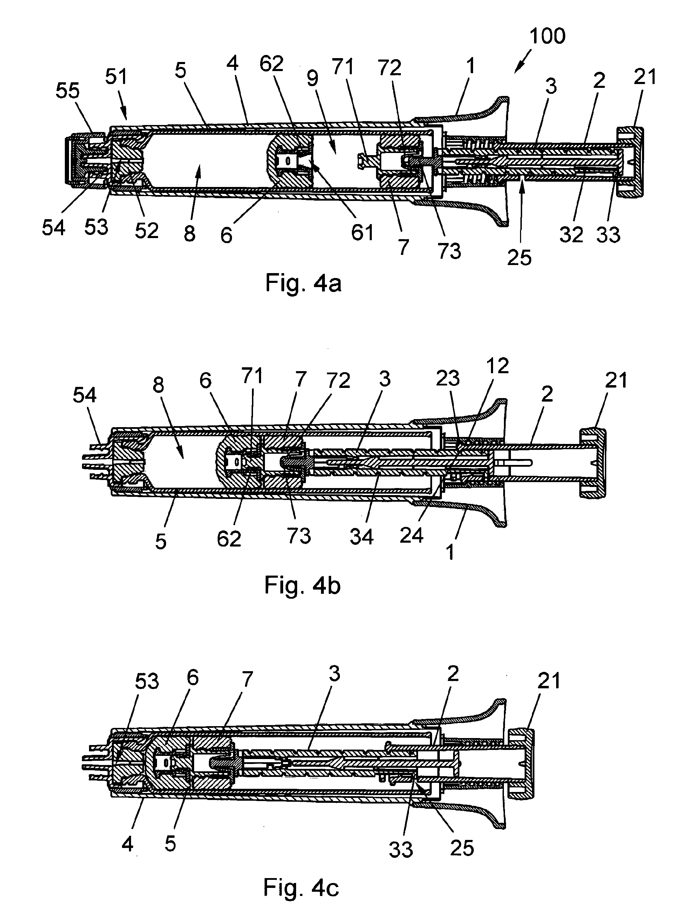

Figs. 4a-4c show a drug delivery device 100 employing the piston rod drive system 10 in different states before and during use.

In Fig. 4a the drug delivery device 100 is in a pre-activation state, i.e. it is shown as delivered by the manufacturer. The main body 1 is pre-connected with a cartridge holder 4 carrying a cartridge 5. The cartridge 5 is of the dual chamber type and it has an outlet end portion 51 sealed by a removable closure 55 and further comprising a plug 52 with a through-going channel 53. The outlet end portion 55 is also provided with a Luer connector 54 for coupling with a suitable delivery member, such as a needle or an infusion set (not shown).

A front piston 6 is arranged in the cartridge 5 just proximally of a conventionally formed bypass section (not visible) in the cartridge wall, i.e. such that at least a portion of the front piston 6 is in sealing contact with the cartridge wall proximally of the bypass section. A front chamber 8 is thereby provided between the outlet end portion 51 and the front piston 6. In the present embodiment the front chamber 8 comprises a dose of powdered, e.g. lyophilised, medicament (not shown). However, in other embodiments the front chamber 8 may comprise a liquid substance. The front piston 6 has a recess 61 in its proximal portion and an insert with radially deflectable arms 62 reaching into the recess 61.

A rear piston 7 is arranged proximally of the front piston 6 and spaced apart therefrom to provide a rear chamber 9 which comprises a solvent (not shown) suitable for reconstitution of the powdered medicament. The rear piston 7 has an insert in its distal portion, which comprises a coupling head 71, and an insert in its proximal portion which comprises radially deflectable arms 72.

The operational portion of the drug delivery device 100 comprises the piston rod drive system 10, which in Fig. 4a is in an initial state corresponding to Fig. 1a. The piston actuation end 31 is in this embodiment connected with a coupling structure 73 which in the shown pre-activation state is axially spaced apart from the rear piston 7. The rear piston 7 is thereby allowed to displace proximally a short distance during storage and/or transport, e.g. due to temperature fluctuations affecting the volume of the solvent in the rear chamber 9, without risking to destroy vital parts of the piston rod drive system 10.

In order to carry out the step of mixing the two substances in the drug delivery device 100 the closure 55 is firstly removed to establish fluid communication to the surroundings. The knob 21 is thereafter turned to advance the driver 2 in the threaded connection with the main body 1. Due to the telescopic arrangement of the piston rod 3, which includes the engagement between the respective longitudinal keyways 35 and the keys 16, the rotation of the driver 2 leads the piston rod 3 outwards in the sleeve 22 in a non-rotational axial movement relative to the main body 1. Initially, the rotation of the driver 2 results in a front portion of the coupling structure 73 being moved past the deflectable arms 72 which lock behind it and thereby provide a harpoon-like coupling between the rear piston 7 and the piston rod 3. By this coupling all subsequent movements of the piston rod 3 are transferred to the rear piston 7.

Continued rotational manipulation of the knob 21 advances the exterior male thread segments 23 further in the interior female thread 12 and the helical motion of the driver 2 relative to the main body 1 brings the knob 21 steadily closer to the main body 1. In this embodiment the ratio of the pitch of the interior female thread 12 to the pitch of the exterior female thread 34 is 1:2.7 which means that on each revolution of the knob 21 the axial displacement of the piston rod 3 relative to the driver 2 is 2.7 times the axial displacement of the driver 2 relative to the main body 1. The axial displacement of the rear piston 7 in the cartridge 5 is therefore more than 2.7 times the axial displacement of the driver 2 in the main body 1 when the knob 21 is operated. During distal displacement of the rear piston 7 the solvent in the rear chamber 9 becomes pressurised and due to its incompressibility it consequently exerts a force on the front piston 6, which moves the front piston 6 into the bypass section (not visible). A free passage is thereby provided for the solvent to bypass the front piston 6 and enter the front chamber 8 as the rear chamber 9 is collapsed. Because of the aforementioned pitch ratio relatively few revolutions of the knob 21 are needed to collapse the rear chamber 9. Nevertheless, as the turning of the knob 21 is naturally executed by the user in discrete steps rather than one continuous motion the speed of advancement of the rear piston 7 during solvent transfer to the front chamber 8 is sufficiently slow to prevent foaming of the mixed product.

Fig. 4b shows the drug delivery device 100 immediately after the completion of solvent transfer and collapse of the rear chamber 9. The front piston 6 and the rear piston 7 are now physically interlocked because the coupling head 71 during the advancement of the rear piston 7 enters the recess 61 and forces itself past the deflectable arms 62 in a manner similar to how the coupling structure 73 initially connects to the rear piston 7. It is noted, however, that this piston interlocking is optional and that in other embodiments of the invention the two pistons merely abut each other upon complete solvent transfer.

Simultaneously with the collapse of the rear chamber 9 the leading face of the exterior male thread segments 23 reaches the rotational stop surface 14 at the end of the interior female thread 12 and the protrusion 33 on the flexible finger 32 snaps into the opening 25 (best seen in Fig. 4c). This provides both a tactile and an audible feedback to the user that the fluid transfer phase has been completed and that the mode of operation of the drug delivery device 100 must be changed.

After having thus transferred the solvent to the front chamber 8 and reconstituted the powdered drug the final administrable product is ready to be delivered. A suitable delivery member, such as an infusion set (not shown), is coupled to the Luer connector 54 and de-aeration of the front chamber 8 may be performed. This can be done in a manner similar to the de-aeration of a conventional syringe because the exterior male thread segments 23 are now out of threaded engagement with the interior female thread 12 and ready to travel the respective axial grooves 15, and the elongated slots 26 are aligned with the keys 16 to guide the driver 2 non-rotationally forward through the main body 1.

Depression of the knob 21 towards the main body 1 will now lead to a common non-rotational axial advancement of the driver 2 and the piston rod 3 in the cartridge 5 and the reconstituted drug will thereby be expelled through the channel 53. Fig. 4c shows the drug delivery device 100 in the end-of-use state after emptying of the cartridge 5.

## Claims

1. A piston rod drive system (10) for a drug delivery device, the piston rod drive system (10) comprising:
- a main body (1) adapted for coupling with a reservoir,
- a user operable activation element (2) operatively coupled with the main body (1) and configured to move axially and rotationally relative to the main body (1) between a first position and a second position,
the piston rod drive system being **characterised by**:
- a piston rod element (3) operatively coupled with the activation element (2) and the main body (1) and configured to move axially and non-rotationally relative to the main body (1) in response to the activation element (2) being moved between the first position and the second position.

2. A system according to claim 1, wherein the activation element (2) is further configured to move axially and non-rotationally relative to the main body (1) between the second position and a third position.

3. A system according to claim 2, wherein the piston rod element (3) is further configured to move axially and non-rotationally relative to the main body (1) in response to the activation element (2) being moved between the second position and the third position.

4. A system according to any of the preceding claims, wherein
- the main body (1) comprises
o a first engagement structure (12) and
o a second engagement structure (16),
- the activation element (2) comprises
o a third engagement structure (23) adapted to cooperate with the first engagement structure (12) to enable a relative rotational motion between the main body (1) and the activation element (2), and
o a fourth engagement structure (24), and
- the piston rod element (3) comprises
o a fifth engagement structure (34) adapted to cooperate with the fourth engagement structure (24) to enable a relative rotational motion between the activation element (2) and the piston rod element (3), and
o a sixth engagement structure (35) adapted to cooperate with the second engagement structure (16) to enable a relative axial and non-rotational motion between the main body (1) and the piston rod element (3).

5. A system according to claim 4, wherein the first engagement structure (12) comprises a first inner thread structure and the third engagement structure (23) comprises a mating first outer thread structure, and wherein the fourth engagement structure (24) comprises a second inner thread structure and the fifth engagement structure (34) comprises a mating second outer thread structure.

6. A system according to claim 5, wherein the pitch of the second outer thread structure is greater than the pitch of the first inner thread structure.

7. A system according to claim 5 or 6, wherein the second engagement structure (16) comprises a key and the sixth engagement structure (35) comprises a keyway.

8. A system according to any of claims 5 - 7, wherein the first inner thread structure terminates in a stop surface (14) structured to abut with a leading face (28) of the first outer thread structure when the activation element (2) and the piston rod element (3) are brought to a specific relative axial position.

9. A system according to claim 8, wherein the stop surface (14) constitutes a portion of an axial groove (15), and wherein the first outer thread structure comprises a circumferentially limited thread segment which is adapted to travel in the axial groove (15) between the stop surface (14) and an end-of-use position to displace the activation element (2) non-rotationally relative to the main body (1).

10. A system according to claim 8 or 9, wherein the activation element (2) comprises a sleeve member (22) adapted to surround at least a portion of the piston rod element (3), and wherein the sleeve member (22) comprises an opening (25) and the piston rod element (3) comprises a flexible arm (32) with a latching protrusion (33) biased to move into the opening (25) when the activation element (2) and the piston rod element (3) reach the specific relative axial position.

11. A drug delivery device comprising:
- a reservoir (5) comprising an outlet (53), a proximal end portion, a first piston (6) arranged between the outlet (53) and the proximal end portion, a second piston (7) arranged between the first piston (6) and the proximal end portion, and a transfer arrangement adapted to enable fluid transfer from one side of the first piston (6) to the other, and
- a piston rod drive system (10) according to any of claims 1 - 10.

12. A drug delivery device comprising:
- a reservoir (5) comprising an outlet (53), a proximal end portion, a first piston (6) arranged between the outlet (53) and the proximal end portion, a second piston (7) arranged between the first piston (6) and the proximal end portion, and a transfer arrangement adapted to enable fluid transfer from one side of the first piston (6) to the other, and
- a piston rod drive system (10) according to claim 3, wherein the main body (1) comprises a first inner thread structure (12) terminating in a stop surface (14), the activation element (2) comprises a first outer thread structure (23) configured for mating engagement with the first inner thread structure (12), and a second inner thread structure (24), and the piston rod element (3) comprises a second outer thread structure (34) configured for mating engagement with the second inner thread structure (24), and wherein the pitch of the first inner thread structure (12) and the pitch of the second inner thread structure (24) are configured such that the piston rod element (3) is displaced axially relative to the main body (1) a distance corresponding to the second piston (7) being brought to abutment or non-releasable engagement with the first piston (6) when a leading face (28) of the first outer thread structure (23) is moved from a pre-activation position into abutment with the stop surface (14).

## Patentansprüche

1. Kolbenstangenantriebssystem (10) für eine Arzneimittelabgabe-vorrichtung, wobei das Kolbenstangenantriebssystem (10) umfasst:
- einen Hauptkörper (1), der zum Koppeln mit einem Behälter angepasst ist,
- ein durch einen Anwender bedienbares Aktivierungselement (2), das mit dem Hauptkörper (1) funktionsfähig gekoppelt ist und konfiguriert ist, dass es sich in Bezug auf den Hauptkörper (1) zwischen einer ersten Position und einer zweiten Position axial und drehbar bewegt,
wobei das Kolbenstangenantriebssystem **gekennzeichnet ist durch**:
- ein Kolbenstangenelement (3), das funktionsfähig mit dem Aktivierungselement (2) und dem Hauptkörper (1) gekoppelt ist und konfiguriert ist, dass es sich als Reaktion auf eine Bewegung des Aktivierungselements (2) zwischen der ersten Position und der zweiten Position in Bezug auf den Hauptkörper (1) axial und nicht drehbar bewegt.

2. System nach Anspruch 1, wobei das Aktivierungselement (2) ferner konfiguriert ist, dass es sich in Bezug auf den Hauptkörper (1) zwischen der zweiten Position und einer dritten Position axial und nicht drehbar bewegt.

3. System nach Anspruch 2, wobei das Kolbenstangenelement (3) ferner konfiguriert ist, dass es sich als Reaktion auf eine Bewegung des Aktivierungselements (2) zwischen der zweiten Position und der dritten Position in Bezug auf den Hauptkörper (1) axial und nicht drehbar bewegt.

4. System nach irgendeinem der vorangehenden Ansprüche, wobei
- der Hauptkörper (1)
o eine erste Eingriffsstruktur (12) und
o eine zweite Eingriffsstruktur (16)
umfasst,
- das Aktivierungselement (2)
o eine dritte Eingriffsstruktur (23), die dazu angepasst ist, mit der ersten Eingriffsstruktur (12) zusammenzuwirken, um eine relative Drehbewegung zwischen dem Hauptkörper (1) und dem Aktivierungselement (2) zu ermöglichen, und
o eine vierte Eingriffsstruktur (24)
umfasst und
- das Kolbenstangenelement (3)
o eine fünfte Eingriffsstruktur (34), die dazu angepasst ist, mit der vierten Eingriffsstruktur (24) zusammenzuwirken, um eine relative Drehbewegung zwischen dem Aktivierungselement (2) und dem Kolbenstangenelement (3) zu ermöglichen, und
o eine sechste Eingriffsstruktur (35), die dazu angepasst ist, mit der zweiten Eingriffsstruktur (16) zusammenzuwirken, um eine relative axiale und nicht drehende Bewegung zwischen dem Hauptkörper (1) und dem Kolbenstangenelement (3) zu ermöglichen,
umfasst.

5. System nach Anspruch 4, wobei die erste Eingriffsstruktur (12) eine erste Innengewindestruktur umfasst und die dritte Eingriffsstruktur (23) eine erste Gegen-Außengewindestruktur umfasst, und wobei die vierte Eingriffsstruktur (24) eine zweite Innengewindestruktur umfasst und die fünfte Eingriffsstruktur (34) eine zweite Gegen-Außengewindestruktur umfasst.

6. System nach Anspruch 5, wobei die Teilung der zweiten Außengewindestruktur größer ist als die Teilung der ersten Innengewindestruktur.

7. System nach Anspruch 5 oder 6, wobei die zweite Eingriffsstruktur (16) einen Keil bzw. Längskeil umfasst und die sechste Eingriffsstruktur (35) eine Keilnut bzw. Aussparung umfasst.

8. System nach irgendeinem der Ansprüche 5-7, wobei die erste Innengewindestruktur in einer Anschlagfläche (14) endet, die strukturiert ist, an einer Führungsfläche (28) der ersten Außengewindestruktur anzuliegen, wenn das Aktivierungselement (2) und das Kolbenstangenelement (3) in eine spezifische relative axiale Position gebracht werden.

9. System nach Anspruch 8, wobei die Anschlagfläche (14) einen Teil einer axialen Auskehlung (15) bildet und wobei die erste Außengewindestruktur ein im Umfang beschränktes Gewindesegment umfasst, das dazu angepasst ist, sich in der axialen Auskehlung (15) zwischen der Anschlagfläche (14) und einer Verwendungsendposition zu bewegen, um das Aktivierungselement (2) in eine Nichtdrehung in Bezug auf den Hauptkörper (1) zu versetzen.

10. System nach Anspruch 8 oder 9, wobei das Aktivierungselement (2) ein Hülsenelement (22) umfasst, das dazu angepasst ist, zumindest einen Teil des Kolbenstangenelements (3) zu umgeben, und wobei das Hülsenelement (22) eine Öffnung (25) umfasst und das Kolbenstangenelement (3) einen beweglichen Arm (32) mit einem Arretierungsvorsprung (33), der vorgespannt ist, um sich in die Öffnung (25) zu bewegen, wenn das Aktivierungselement (2) und das Kolbenstangenelement (3) die spezifische relative axiale Position erreichen, umfasst.

11. Arzneimittelabgabevorrichtung, umfassend:
- einen Behälter (5), umfassend einen Auslass (53), einen proximalen Endabschnitt bzw. -teil, einen ersten Kolben (6), der zwischen dem Auslass (53) und dem proximalen Endabschnitt angeordnet ist, einen zweiten Kolben (7), der zwischen dem ersten Kolben (6) und dem proximalen Endabschnitt angeordnet ist, und eine Übertragungsanordnung, die zum Ermöglichen einer Fluidübertragung von einer Seite des ersten Kolbens (6) zur anderen angepasst ist, und
- ein Kolbenstangenantriebssystem (10) nach irgendeinem der Ansprüche 1-10.

12. Arzneimittelabgabevorrichtung, umfassend:
- einen Behälter (5), umfassend einen Auslass (53), einen proximalen Endabschnitt bzw. -teil, einen ersten Kolben (6), der zwischen dem Auslass (53) und dem proximalen Endabschnitt angeordnet ist, einen zweiten Kolben (7), der zwischen dem ersten Kolben (6) und dem proximalen Endabschnitt angeordnet ist, und eine Übertragungsanordnung, die zum Ermöglichen einer Fluidübertragung von einer Seite des ersten Kolbens (6) zur anderen angepasst ist, und
- ein Kolbenstangenantriebssystem (10) nach Anspruch 3, wobei der Hauptkörper (1) eine erste in einer Anschlagfläche (14) endende Innengewindestruktur (12) umfasst, das Aktivierungselement (2) eine erste zum Gegeneingriff mit der ersten Innengewindestruktur (12) konfigurierte Außengewindestruktur (23) und eine zweite Innengewindestruktur (24) umfasst und das Kolbenstangenelement (3) eine zweite zum Gegeneingriff mit der zweiten Innengewindestruktur (24) konfigurierte Außengewindestruktur (34) umfasst, und wobei die Teilung der ersten Innengewindestruktur (12) und die Teilung der zweiten Innengewindestruktur (24) derart konfiguriert sind, dass das Kolbenstangenelement (3) axial in Bezug auf den Hauptkörper (1) um einen Abstand versetzt wird, der dem zum Anliegen an den oder in nicht lösbaren Eingriff mit dem ersten Kolben (6) gebrachten zweiten Kolben (7), wenn eine Führungsfläche (28) der ersten Außengewindestruktur (23) von einer Voraktivierungsposition zum Anliegen an die Anschlagfläche (14) bewegt wird, entspricht.

## Revendications

1. Un système d'entrainement de tige de piston (10) pour un dispositif de délivrance de médicament, le système d'entrainement de tige de piston (10) comprenant :
- un corps principal (1) apte à être couplé à un réservoir,
- un élément d'activation manoeuvrable par l'utilisateur (2) couplé de manière opérante au corps principal (1) et configuré pour se déplacer axialement et en rotation par rapport au corps principal (1) entre une première position et une seconde position,
le système d'entrainement de tige de piston étant **caractérisé par** :
- un élément de tige de piston (3) couplé de manière opérante à l'élément d'activation (2) et au corps principal (1) et configuré pour se déplacer axialement et non en rotation par rapport au corps principal (1) en réponse au déplacement de l'élément d'activation (2) entre la première position et la seconde position.

2. Un système selon la revendication 1, dans lequel l'élément d'activation (2) est en outre configuré pour se déplacer axialement et non en rotation par rapport au corps principal entre la seconde position et une troisième position.

3. Un système selon la revendication 2, dans lequel l'élément de tige de piston (3) est en outre configuré pour se déplacer axialement et non en rotation par rapport au corps principal (1) en réponse au déplacement de l'élément d'activation (2) entre la seconde position et la troisième position.

4. Un système selon l'une des revendications précédentes, dans lequel :
- le corps principal (1) comprend :
. une première structure de venue en prise (12) et
. une seconde structure de venue en prise (16),
- l'élément d'activation (2) comprend :
. une troisième structure de venue en prise (23) apte à coopérer avec la première structure de venue en prise (12) pour autoriser un mouvement en rotation relative entre le corps principal (1) et l'élément d'activation (2), et
. une quatrième structure de venue en prise (24), et
- l'élément de tige de piston (3) comprend :
. une cinquième structure de venue en prise (34) apte à coopérer avec la quatrième structure de venue en prise (24) pour autoriser un mouvement en rotation relative entre l'élément d'activation (2) et l'élément de tige de piston (3), et
. une sixième structure de venue en prise (35) apte à coopérer avec la seconde structure de venue en prise (16) pour autoriser un mouvement axial et non en rotation entre le corps principal (1) et l'élément de tige de piston (3).

5. Un système selon la revendication 4, dans lequel la première structure de venue en prise (12) comprend une première structure taraudée et la troisième structure de venue en prise (23) comprend une première structure filetée conjuguée, et dans lequel la quatrième structure de venue en prise (24) comprend une seconde structure taraudée et la cinquième structure de venue en prise (34) comprend une seconde structure filetée conjuguée.

6. Un système selon la revendication 5, dans lequel le pas de la seconde structure filetée est supérieur au pas de la première structure taraudée.

7. Un système selon la revendication 5 ou 6, dans lequel la seconde structure de venue en prise (16) comprend une clavette et la sixième structure de venue en prise (35) comprend une cannelure.

8. Un système selon l'une des revendications 5 à 7, dans lequel la première structure taraudée se termine par une surface d'arrêt (14) structurée pour venir en butée contre une face frontale (28) de la première structure filetée lorsque l'élément d'activation (2) et l'élément de tige de piston (3) sont amenés dans une position axiale relative spécifique.

9. Un système selon la revendication 8, dans lequel la surface d'arrêt (14) constitue une partie d'une gorge axiale (15), et dans lequel la première structure filetée comprend un segment fileté limité circonférentiellement qui est apte à se déplacer dans la gorge axiale (15) entre la surface d'arrêt (14) et une position de fin d'utilisation pour déplacer l'élément d'activation (2) sans rotation par rapport au corps principal (1).

10. Un système selon la revendication 8 ou 9, dans lequel l'élément d'activation (2) comprend un organe de manchon apte à entourer au moins une partie de l'élément de tige de piston (3), et dans lequel l'organe de manchon (22) comprend une ouverture (25) et l'élément de tige de piston (3) comprend un bras flexible (32) avec une saillie de verrouillage (33) sollicitée de manière à se déplacer dans l'ouverture (25) lorsque l'élément d'activation (2) et l'élément de tige de piston (3) atteignent la position axiale relative spécifique.

11. Un dispositif de délivrance de médicament, comprenant :
- un réservoir (5) comprenant un orifice de sortie (53), une partie d'extrémité proximale, un premier piston (6) disposé entre l'orifice de sortie (53) et la partie d'extrémité proximale, un second piston (7) disposé entre le premier piston (6) et la partie d'extrémité proximale, et une disposition de transfert apte à permettre un transfert de fluide d'un côté du premier piston (6) vers l'autre, et
- un système d'entrainement de tige de piston (10) selon l'une des revendications 1 à 10.

12. Un dispositif de délivrance de médicament comprenant :
- un réservoir (5) comprenant un orifice de sortie (53), une partie d'extrémité proximale, un premier piston (6) disposé entre l'orifice de sortie (53) et la partie d'extrémité proximale, un second piston (7) disposé entre le premier piston (6) et la partie d'extrémité proximale, et une disposition de transfert apte à permettre un transfert de fluide d'un côté du premier piston (6) vers l'autre, et
- un système d'entrainement de tige de piston (10) selon la revendication 3, dans lequel le corps principal (1) comprend une première structure taraudée (12) se terminant dans une surface d'arrêt (14), l'élément d'activation (2) comprend une première structure filetée (23) configurée pour venir en prise conjuguée avec la première structure taraudée (12), et une seconde structure taraudée (24), et l'élément de tige de piston (3) comprend une seconde structure filetée (34) configurée pour venir en prise conjuguée avec la seconde structure taraudée (24), et dans lequel le pas de la première structure taraudée (12) et le pas de la seconde structure taraudée (24) sont configurés de telle manière que l'élément de tige de piston (3) soit déplacé axialement par rapport au corps principal (1) sur une distance correspondant à l'amenée du second piston (7) en butée ou en prise non libérable avec le premier piston (6) lorsqu'une face frontale (28) de la première structure filetée (23) est déplacée d'une position de pré-activation vers la venue en butée avec la surface d'arrêt (14).
